# EUROPEAN PATENT APPLICATION

(11) **EP 3 862 325 A1**
(43) Date of publication of application: **11.08.2021**
(21) Application number: 19887866.2
(22) Date of filing: 19.11.2019
(51) Int. Cl.: C02F 1/461, C02F 1/467, A61L 2/18, A61L 2/22, A01K 1/00

(54) **STERILIZATION ELECTRODE, METHOD FOR MANUFACTURING SAME, AND STERILIZATION APPARATUS USING SAME**

(30) Priority: 21.11.2018 KR 20180144912
(71) Applicant: Lee, Yeanju, Gyeonggi-do 13423 (KR)
(72) Inventor: Lee, Yeanju, Gyeonggi-do 13423 (KR)
(74) Representative: Stolmár & Partner Patentanwälte PartG mbB
(86) International application number: PCT/KR2019/015822
(87) International publication number: WO 2020/106006

(57) **Abstract**

The present invention relates to a set of sterilizing electrodes, to a set of sterilizing electrodes, method of sterilizing and sterilization apparatus using same, comprising: a conductive body formed of electrically conductive material and having a facing surface and connected to power supply; an electrode catalyst layer stacked on the facing surface of the conductive body; and, an insulation cover formed of an insulating material, and is fixed to the conductive body with the electrode catalyst layer interposed between the conductive body, wherein through portions are provided to form a first region that is a exposed part of the electrode catalyst layer through the through portions at a plurality of positions, and wherein the remaining areas of the electrode catalyst layer other than the first region are covered by the insulation cover; thereby obtaining advantageous effects that it is easy to manufacture to replace and to recycle the electrode catalyst layer, and also sterilizing components can be uniformly and effectively generated.

## Description

### TECHNICAL FIELD

The present invention disclosed herein relates to a set of sterilizing electrodes, method of sterilizing and sterilization apparatus using same, and more particularly, to a set of sterilizing electrodes, method of sterilizing and sterilization apparatus using same for being capable of continuously producing a large amount of sterilizing water such as for livestock barn by supplying a high current for a long time.

### BACKGROUD OF THE INVENTION

Recently, various pathogens have been generated, and pathogens are introduced into the livestock barns by the wind, and thus to cause serious problems of infection or death of livestock such as cattle, sheep, pigs, horses, poultry, e.g., chickens, ducks, pheasants and horses raised in the livestock barns.

Moreover, livestock raised in the barn live in a state where a large number of animals is accommodated compared to the area. Accordingly, when some livestock or poultry in the barn is infected with pathogens, other livestock or poultry inside the barn are easily infected.

Accordingly, there is a need to keep the inside of the barn in a cleaner environment and remove pathogens immediately when pathogens are introduced from the outside into the barn, thereby minimizing the infection of livestock and poultry inside the barn.

According to Korean Patent No. 10-1612920, only the air that has passed through the filter is introduced into the inside of the barn thereby preventing contaminants from flowing into the barn, while the humidity inside the barn is controlled by controlling the temperature inside the barn.

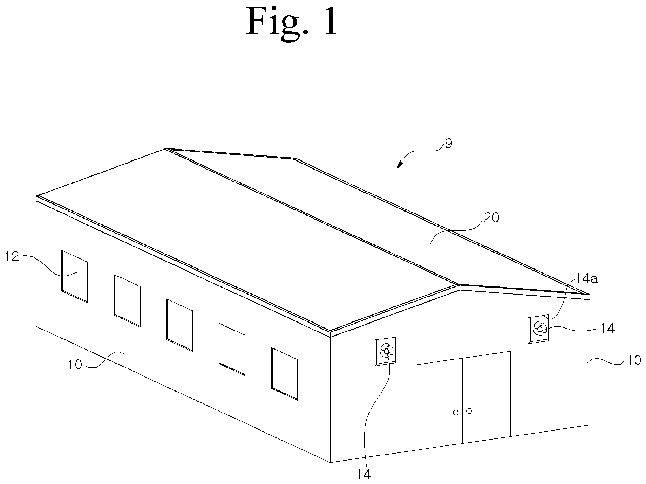

However, even with the above configuration, as shown in Fig. 1, it is only possible to suppress the inflow of pollutants including pathogens in the outside atmosphere into the barn only when the barn 9 is formed in a closed form from the outside. In other words, under the condition that the barn 9 should be blocked in all directions by the side wall 10 and should be blocked in upper direction by the roof 20, and also all the outside air should pass the ventilation fan 14 to the inside of the barn 9, the pollutant such as pathogens cannot be introduced into the barn 9.

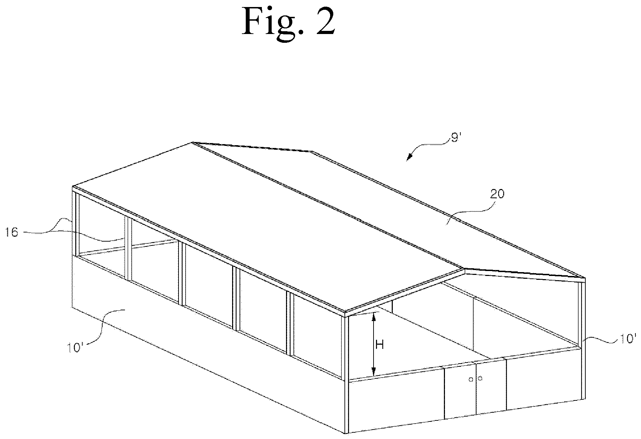

However, as shown in Fig. 2, open barns are widely and generally used. In the open-type barn, as the roof 20 is connected to the side wall 10' only by a predetermined height H of the pillar 16, a passage is provided between the roof 20 and the side wall 10' whereby it is easy for outside air to enter into the inside of the barn 9'. Therefore, the above construction cannot be applied to the open-type barn 9'.

In addition, in the open-type barn 9', the passage between the roof 20 and the side wall 10' may be blocked between the inside and the outside by spreading the vinyl film rolled from the roof 20 downwards in rainy or cold weather. However, it has a limitation in that the spread vinyl film cannot completely prevent external air from being introduced into the inside of the barn.

Moreover, in the closed-type barn 9, as the air flow between the inside and the outside is limited, the excrement of livestock or poultry in the barns may cause a bigger contamination problem than the open-type barn 9'.

Therefore, irrespective of the type of barn such as closed-type or open-type, there is an urgent need to prevent the infection of pathogens such as foot-and-mouth disease or avian flu virus to livestock and poultry by sufficiently sterilizing the inside of the barn.

On the other hand, contamination problems by microorganisms and bacteria have been generated in facilities widely used in daily life such as kitchen utensils, bidets, showers, and tooth scrubbers for cleaning teeth by spraying water under high pressure.

Kitchen utensils such as pots, knives, spoons, and cutting boards may be contaminated by various germs if they are not sufficiently washed after contact with food. When cooking food with contaminated kitchen utensils or eating food contained in contaminated kitchen utensils, anyone who eats the food may be infected by the germ. Further, it is widely known that the water jetting tube of bidets used in toilets or the interior of the shower head of the shower facilities may be easily contaminated.

Similarly, even in tooth scrubbers, e.g., waterpik, that spray water under high pressure to remove foreign substances stuck between teeth, contamination of tubes for supplying water under high pressure has been discussed as a hygiene problem.

In order to solve such a contamination problem, there is an urgent need for a method of removing germs, bacteria, pathogens causing contamination or infection by manufacturing and supplying a sufficient amount of sterilized water to them in a short time.

### SUMMARY OF INVENTION

In order to solve the above problems, an object of the present invention is to provide a set of sterilizing electrodes for producing a large amount of sterilizing water in a short time, a method for producing the same, and a sterilization apparatus using the same.

Above all, the present invention has an object to reliably produce sterilized water by always maintaining a constant concentration of sterilizing components such as hypochlorous acid in the sterilized water for tens or more hours, even if the sterilized water is manufactured by electrolyzing water containing chlorine.

In addition, the present invention has an object to generate a sterilizing component having a high sterilization ability while minimizing the power consumption and the consumption of electrode catalysts such as platinum by more efficient electrolysis of liquid with forming a plurality of separate electricity paths between the negative electrode and the positive electrode facing each other.

In particular, the present invention has an object to provide a structure in which a platinum catalyst layer is simply formed as having a thick thickness than a plating layer on at least one of the set of sterilizing electrodes, so that a large amount of sterilizing water can be continuously generated by electrolysis without replacing the electrode for a long time.

That is, the present invention has an object to easily and simply manufacture a set of sterilizing electrodes having a thick electrode catalyst layer.

Also, the present invention has an object to continuously produce a large amount of sterilized water for a long time.

In addition, the present invention has an object to improve economic efficiency by reusing the unused portion of the electrode catalyst layer made of expensive platinum.

The present invention has an object to provide a sterilization apparatus that may be applied to both a closed-type barn and an open-type barn by using the above-mentioned set of sterilizing electrodes.

The present invention has an object to effectively sterilize the inside of a livestock barn, even for the inside of the well-ventilated barn.

The present invention has an object to sterilize various objects such as tooth scrubbers, bidets, water supply pipes of shower facilities, kitchen utensils, etc. using the sterilizing water produced in a large amount in a short period of time by using the set of sterilizing electrodes manufactured as described above.

In order to achieve the objects of the present invention, a set of sterilizing electrodes is provided with including a first electrode and a second electrode disposed to face each other for generating at least one sterilizing component in a liquid containing chlorine between the first electrode and the second electrode by supplying power to the first electrode and the second electrode, wherein at least one of the first electrode and the second electrode comprises: a conductive body including an electrically conductive material for being connected to the power source; an electrode catalyst layer formed by stacking electrode catalyst on a facing surface of the conductive body; an insulating cover fixed to cover the outer surface of the electrode catalyst layer with the electrode catalyst layer interposed between the conductive body, wherein a plurality of through portions are formed in the insulating cover so that a first region of a part of the electrode catalyst layer is exposed at a plurality of positions through the through portions and the remaining region of the other part of the electrode catalyst layer is covered.

Therefore, the first region of the electrode is formed by the through portions of the insulating cover covering the electrode catalyst layer, and a plurality of electricity conduction paths between the first electrode and the second electrode are formed to include the first region, so that it is possible for a plurality of electricity conduction paths to be more easily and definitely formed.

Above all, the electrode catalyst layer may be formed as a form of a thin plate and be combined or assembled by stacking on the facing surface of the electrode. For example, the electrode catalyst layer may be formed of a thin plate having a thickness of 20 µm to 3 mm, and it is possible to form much thicker than 3 µm of the thickness of a general platinum plating layer.

In other words, in conventional electrodes, as platinum Pt is formed integrally on the facing surface of the electrode with a thin plating layer of about 3 µm. Thus, when a low current of about 300 to 500 mA is applied for 15 minutes a day for 30 days for generating sterilizing component, there is a problem that electrolysis can no longer be lasted with the conventional electrodes. In addition, when a high current of 10A or more is applied, there is a serious problem that the electrolysis can be continued only for a period of less than 1 hour. However, in the present invention, as a sufficiently thick platinum body is stacked in the first region 120A as the electrode catalyst layer in the form of a lump or a plate, even if a high current of 10A or more (for example, 30A to 40A) is applied to the electrode, it is possible to obtain operational reliability capable of continuously performing electrolysis for a period of 10 days or more.

In other words, it is possible to form the electrode catalyst layer much thicker than plating layer, so that even if a much higher electricity current is supplied to the electrodes for a long time, it is possible to reliably and continuously manufacture sterilized water by the electrolysis for a long time.

In addition, as the electrode catalyst layer is formed as a thin plate and is stacked on the facing surface of the conductive body instead of being attached thereon, when the platinum exposed in the first region is used up by the electrolysis, by separating the insulating cover from the conductive body, platinum remaining in the area except for the first region can be used for subsequent electrolysis. Since platinum has very good ductility, the used platinum from the electrode catalyst layer can be recycled to a sterilizing electrode by forming a new electrode catalyst layer with additional platinum having the original thickness or a new thinner electrode catalyst layer without the additional platinum.

To this end, it is preferable that the insulating cover is detachably coupled to the conductive body, and the electrode catalyst layer is also detachably placed on the conductive body. For example, the insulating cover may be assembled by fitting to the conductive body with interposing the electrode catalyst layer therebetween. Or the insulating cover may be assembled to the conductive body by fastening bolts or the like. That is, the insulating cover plays a role in fixing the position of the electrode catalyst layer and in exposing the electrode catalyst layer with the through portions to outside only in the first region to form the separate electricity current paths.

For example, the insulating cover may be formed of any one of plastic, resin, polyurethane, and rubber.

On the other hand, the electrode catalyst layer is made of a platinum-plated layer on the facing surface of the electrode, and the insulating cover is provided with a surface formed in lattice shapes, and thus the first region may be formed by the through portion of the lattice. As it is impossible to separate the electrode catalyst layer from the conductive body, it is disadvantageous not to reuse the electrode catalyst layer. However, as a plurality of electricity current path may be formed without forming the conductive body into a complex shape, it is advantageous to simply manufacture the first region to form the plurality of electricity current paths.

Meanwhile, the conductive body may be formed of titanium, copper, or carbon having excellent electrical conductivity.

In addition, the original insulating cover may be formed to be replaceable with another insulating cover in which through portions are formed at different positions from the original insulating cover. Thus, when the first region of the electrode catalyst layer is consumed by more than a predetermined amount by electrolysis, the original insulating cover may be replaced with another insulating cover having through portions at different positions.

The first electrode and the second electrode may be formed to include the conductive body, the insulating cover, and the electrode catalyst layer shaped as a thin plate. Herein, a first region of the first electrode and another first region of the second electrode are aligned to each other so that a plurality of electricity current paths are separately formed with one another between the first region of the first electrode and the first region of the second electrode.

That is, when the first region of the first electrode and another first region of the second electrode are installed to face each other, and when electricity current is supplied to the electrode catalyst layer of each electrode, the electric charges of the current are concentrated on the first region of the electrode catalyst layer of the electrodes and plural electricity current paths are formed for the electric charges to move from one first region to another first region of the electrodes facing each other and thus to cause electrolysis. In this way, as electric charges are conducted in a plurality of electric paths between the first regions facing each other, sterilizing water containing sterilizing components such as oxidizing material and hypochlorous acid is generated. Also, as the sterilizing components are generated in the plural electricity current paths, the amount of the sterilizing components may be precisely controlled and the amount thereof is increased at the same time.

For example, the sterilizing electrodes may be used for producing sterilizing water to be supplied to livestock barns.

On the other hand, the present invention provides a method of manufacturing sterilizing electrode, comprising: preparing a conductive body formed of electrically conducting material and has a facing surface; stacking an electrode catalyst layer formed as a sheet having a predetermined thickness on the facing surface of the conductive body; and fixing an insulating cover having a plurality of through portions with the electrode catalyst layer interposed between the conductive body; wherein a part of the electrode catalyst layer is exposed to form a first region at plural positions through the through portions.

Herein, in fixing the insulating cover, it is preferable to detachably fix the insulating cover to the conductive body. Accordingly, when the platinum consumption in the first region exceeds the reference value, the electrode catalyst layer may be replaced. For example, the electrode catalyst layer may be replaced with a new insulating cover having the through portions at different positions so as to continue electrolysis with the unused part of the used electrode catalyst layer.

Here, the insulating cover may be formed in a lattice shape, and the through portions of the lattice may form the above first region. In this case, as a plurality of electricity current paths are formed between the first electrode and as the plurality of electricity current paths are uniformly separated with one another, the amount of sterilizing components such as hypochlorous may be further increased.

On the other hand, the present invention provides a sterilization apparatus for generating a liquid containing an electrolytic material into sterilizing water containing sterilizing components by supplying electricity current from a power supply to the set of sterilizing electrodes having the above-described configuration.

As described above, as electrolysis is performed in the current path between the first region of the electrode catalyst layer which is sufficiently thick and detachable, sterilizing components as oxidants like hypochlorous acid HOCl, OH radicals, peroxide H₂O₂, etc. are generated by electrolysis in the liquid between the first electrode and the second electrode, and the liquid is made into sterilizing water. For this purpose, it is preferable that the liquid contains a chlorine Cl. Further, since hypochlorous acid is harmless to the human body and has high sterilizing capability, in order to increasingly produce the amount of hypochlorous acid as much as possible, the pH value of the liquid is preferably in the range of 3.0 to 8.0.

According to another aspect of the present invention, the first electrode and the second electrode may be formed to include the conductive body, the insulating cover, and the electrode catalyst layer. The first electrode and the second electrode are disposed such that the first region formed by the through portions of the insulating cover are aligned to face each other between the first electrode and the second electrode.

A sterilizing water supply unit may be further comprised for supplying sterilizing water with the sterilizing component whereby the sterilizing water may be supplied to various objects to be sterilized.

For example, the sterilizing water supply unit includes a plurality of supply bars with a plurality of spray ports, and the supply bars are installed in a plurality of rows under the ceiling of the barn, so that the sterilizing water can be supplied from the spray port to sterilize the interior of the barn.

In addition, the sterilizing electrode for generating an oxidizer may be installed in the still liquid to generate a sterilizing component in the still liquid. However, the present invention is not limited thereto. According to another aspect of the present invention, the set of sterilizing electrodes having the first electrode and the second electrode as a pair may be installed in a flowing liquid.

That is, the receiving portion is disposed for accommodating the set of sterilizing electrodes in the middle of the supply pipe for supplying the liquid, and the sterilizing components are generated in real time in the flowing liquid through the supply pipe by the set of sterilizing electrodes, and sterilizing water containing the sterilizing component may be supplied to an object to be sterilized, for example, a tooth cleaner, a water pipe, a bidet, a water purifier, and the like.

Here, the receiving portion in which the set of sterilizing electrodes are disposed may be positioned in the sterilization region ST where the flow cross section of the liquid flow is larger than that of neighboring regions. Thus, as the flow velocity of the liquid passing through the set of sterilizing electrodes is lowered, the sterilizing components generated by the electrodes may be contained in larger amount in the flowing liquid.

In addition, the sterilization region ST may be disposed between bent portions of the pipe through which the liquid flows, so that the velocity of the liquid flow is reduced due to friction caused by a change in the flow direction at the bend portions whereby more sterilizing components are contained in the flowing liquid to make it possible to produce higher sterilizing water in real time.

Here, since the electrode catalyst layer made of platinum is formed as a thin plate which is much thicker than the plating layer in prior arts, even if a high current is applied to the electrodes, the platinum can be maintained for a long time to continue the electrolysis. Accordingly, a high current of 10 ampere (A) or more, e.g., 10A to 50A, may be allowed to be applied to the sterilizing electrodes.

The sterilization apparatus configured as described above can be applied in various ways. That is, the sterilization apparatus has the advantage that the amount of sterilization components produced by the current or electric charges between the first regions of the sterilizing electrodes facing each other is uniformly controlled, so that the amount of sterilization components produced per unit time can be maintained as constant. Thus, the sterilization apparatus may be used for objects to be required to supply a large amount of sterilized water having an overall uniform concentration distribution in a short time, it can be used for various purposes.

First, the sterilizing water produced by the sterilizing apparatus having the above-described configuration may be supplied to the livestock barn by the sterilizing water supply unit. Since the sterilization apparatus can manufacture a large amount of sterilized water while maintaining a reliable range of concentration for a long period of time, sterilizing water is continuously supplied to a wide barn, thereby killing pathogens or the like in the barn and maintaining it as a sanitary barn.

In particular, since it does not sterilize the air flowing into the barn, even if pathogens or the like are introduced into the barn, a sufficient amount of sterilizing water can be sprayed into the air to obtain an advantageous effect of effectively sterilizing a large space inside the barn.

For example, in the sterilization water supply unit that supplies sterilizing water inside the barn, plural supply bars equipped with a plurality of spraying ports are arranged at intervals in a plurality of rows under the ceiling of the barn, so that the sterilizing water is sprayed evenly in the interior space of the barn thereby maintaining a clean and hygienic environment.

In addition, the sterilizing apparatus configured as described above may be used for sterilizing and cleaning a liquid transfer pipe. That is, the inside of the liquid transfer tube can be sterilized by passing the sterilizing water through the liquid transfer tube, wherein the sterilizing water is produced by the sterilization apparatus configured as described above.

For example, it may be used for sterilizing and cleaning the inner wall of a tooth cleaner such as Waterpik, or a liquid transfer pipe for transferring water to a bidet, a water pipe, or a water purifier.

In addition, it can be used to clean kitchen utensils, knives, and cutting boards used in the kitchen. That is, as the sterilization apparatus according to the present invention is capable of producing a large amount of sterilizing water in a short time and maintaining the ratio of sterilizing components in a reliable range for a long time, the sterilizing water may be used except for the sensitive parts such as eye of human body, the inside of nose or mucous membrane.

As described above, according to the present invention, there is provided a set of sterilizing electrodes for forming a plurality of current paths to be spaced apart from each other by a first region formed by an insulating cover that exposes only a part of an electrode catalyst layer.

Above all, the present invention obtains an advantageous effect that the thickness of the electrode catalyst layer can be formed much thicker than that of the plating layer, and thus the water containing chlorine may be continuously electrolyzed for a long time to produce sterilizing water containing sterilizing components, thereby reliably generating sterilizing water that uniformly keeps the concentration of hypochlorous acid in the sterilizing water constant.

The present invention obtains an advantageous effect that, as the negative electrode and the positive electrode facing each other form a plurality of current paths, sterilizing components are generated with a uniform concentration in the liquid by the electrolysis on the current paths.

That is, the present invention realizes that the negative electrode and the positive electrode facing each other form a plurality of current paths, so that the concentration of the sterilizing components can be precisely controlled.

In particular, in the present invention, as the electrode catalyst layer may be stacked on the facing surface of the conductive body, and as the electrode catalyst layer may be formed to have a larger thickness which cannot be formed by plating, it is possible to obtain the effect of continuously and reliably generating a large amount of sterilizing components per unit time for a long time even if higher current may be supplied to the electrodes.

Moreover, in the present invention, as the electrode catalyst layer is formed to be stacked on the facing surface of the conductive body, when the electrode catalyst layer the first region is consumed in which the current paths in liquid are formed, the used electrode catalyst layer may be simply replaced with a new electrode catalyst layer, and the used catalyst layer is easily separated and recycled, thereby increasing economic efficiency by using up all of the expensive platinum.

In addition, the present invention is formed so as to be replaceable with another insulating cover in which the through portions are arranged differently, so that all unused catalysts can be sequentially used up without replacing the electrode catalyst layer thereby obtaining an effect of increasing economic feasibility.

According to the present invention, the electrode catalyst layer thicker than the plating layer may be stacked on the facing surface of the sterilizing electrode without adhesion or adhesion, so that the thickness of the electrode catalyst layer is freely formed, and thus a large amount by electrolysis without replacing the electrode for a long time may continuously performed to manufacturing sterilized water to be supplied to livestock barns.

The present invention can obtain an advantageous effect of easily and simply manufacturing a sterilizing electrode having a thick electrode catalyst layer.

The present invention can obtain an advantageous effect of generating the sterilizing components with a predetermined concentration even in a liquid flow field.

The present invention can obtain the effect of maintaining a hygienic and clean environment not only for closed-type livestock barns or houses but also for open-type livestock barns or houses by using sterilizing water produced in a large amount within a short time.

In addition, according to the present invention, it is possible to obtain an advantageous effect of being able to cleanly sterilize the inside of a pipe line of various devices such as a bidet supplying liquid, a tooth cleaner, and a water purifier.

### BRIEF DESCRIPTION OF THE DRAWINGS

The accompanying drawings are included to provide a further understanding of the present invention, and are incorporated in and constitute a part of this specification. The drawings illustrate exemplary embodiments of the present invention and, together with the description, serve to explain principles of the present invention. In the drawings:
Fig. 1 is a perspective view showing the external shape of a closed-type barn.
Fig. 2 is a perspective view showing the external shape of the open-type barn.
Fig. 3 is a flowchart sequentially showing a method of manufacturing a sterilizing electrode according to one embodiment of the present invention.
Fig. 4 is an exploded perspective view of the sterilizing electrode.
Fig. 5 is a perspective view of the sterilizing electrode of Fig. 4.
Fig. 6 is a view showing the configuration of a sterilization apparatus according to one embodiment of the present invention.
Fig. 7 is an enlarged view of part 'A' of Fig. 6.
Fig. 8 is a view showing the configuration of a sterilization apparatus according to another embodiment of the present invention.
Fig. 9A is a plan view of Fig. 5;
Fig. 9B is a plan view of a configuration in which the insulating cover is removed while electrode platinum catalyst layer as a form of thin plate in the first region of the sterilizing electrode of FIG. 9A is consumed.
Fig. 9C is a plan view of a sterilizing electrode replaced with another insulating cover in which through portions at different positions are formed.
Fig. 10 is a view showing the configuration of a sterilization apparatus using the sets of sterilizing electrodes according to the present invention.
Fig. 11 is an enlarged view of the sets of sterilizing electrodes receiving portion ST of Fig. 10.
Fig. 12 is an exploded view showing the inside configuration of the set of electrodes of the sterilizing apparatus according to another embodiment of the present invention.
Fig. 13 is a view for explaining the operation of the sterilization apparatus using the electrode of Fig. 12;
Fig. 14 is a graph showing a comparison graph between the results of a test result of the amount of the produced sterilizing components produced using the electrode of Fig. 13 and the test result of the amount of the produced sterilizing components using existing electrodes in prior art.
Fig. 15 is a schematic perspective view of a livestock sterilization apparatus using the sterilization apparatus of Fig. 10 for sterilization of a livestock barn.

### DETAILED DESCRIPTION OF PREFERRED EMBODIMENTS

Preferred embodiments of the present invention will be described below in more detail with reference to the accompanying drawings. The present invention may, however, be embodied in different forms and should not be constructed as limited to the embodiments set forth herein. Rather, these embodiments are provided so that this disclosure will be thorough and complete, and will fully convey the scope of the present invention to those skilled in the art.

Hereinafter, it will be described about an exemplary embodiment of the present invention in conjunction with the accompanying drawings.

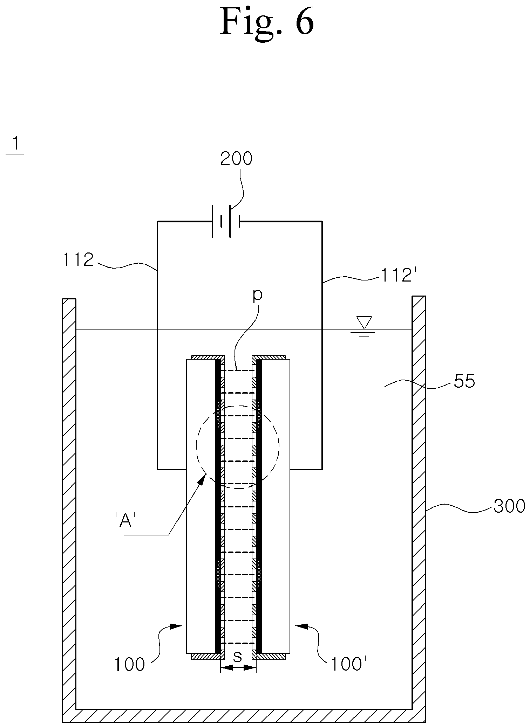

As shown in Fig. 6, the sterilization apparatus according to the present invention comprises a set of sterilizing electrodes including a first electrode 100 and a second electrode 100' which are disposed to face each other at least partially immersed in liquid 55 containing electrolytic material such as salt or chlorine, etc. Here, the liquid 55 is accommodated in a receiving container 300. Here, the set of sterilizing electrodes are supplied with power from the power supply unit 200 to the first electrode 100 and the second electrode 100' to generate sterilizing components such as hypochlorous acid in the liquid 55.

Here, the first electrode 100 is connected to the anode from the power supply unit 200, the second electrode 200 is connected to the cathode of the power supply unit 200, and thus electricity current is supplied to the set of sterilizing electrodes.

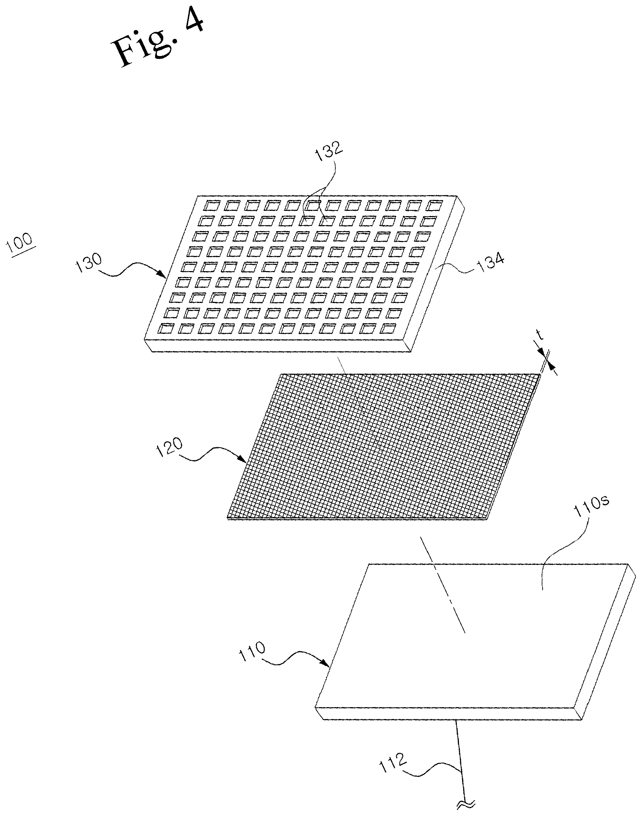

At least one of the first electrode 100 and the second electrode 100' includes a conductive body 110, an electrode catalyst layer 120, and an insulating cover 130. Although the first electrode 100 is shown in FIG. 4 for convenience, the second electrode 100' shown in FIG. 6 may be configured in the same manner as the first electrode, the description of the second electrode 100' will be replaced with the description of the first electrode 100.

The conductive body 110 is formed of an electrically conductive material having excellent electrical conductivity, such as titanium or copper, and has a facing surface 110s. In addition, the conductive body 110 is connected with the power supply line 112 extended from the power supply unit 200.

Here, the facing surface 110s refers to a surface facing the electrode catalyst layer 120' of another electrode facing each other, and may be formed as a flat surface as shown in the drawing or a curved surface.

The electrode catalyst layer 120 is preferably made of platinum in the form of a plate, and may be formed of iridium Ir which is an oxygen reaction catalyst, or may be formed of cobalt or nickel. That is, the material forming the electrode catalyst layer 120 may include any one of platinum, cobalt, iridium, and nickel.

Here, the molecular structure of the electrode catalyst may be formed in a structure in which any one of platinum, cobalt, iridium, and nickel is disposed in the center and graphene is coated on the surface thereof. Through this, it is possible to obtain an advantage of increasing the electrolysis efficiency while reducing the consumption amount of the electrode catalyst.

Hereinafter, the electrode catalyst layer 120 made of a platinum material, which has high ductility and is easily shaped into a thin plate, will be described as an example.

According to an embodiment of the present invention, the electrode catalyst layer 120 may be formed on the facing surface 110s of the conductive body 110 by plating, but the thickness of the electrode catalyst layer formed by plating is very thin and thus there is a limit to use for a long time. However, even when the electrode catalyst layer 120 is formed by plating, as will be described later, the advantage effect may be obtained that the first region 120A forming a plurality of electricity current paths p by the through portions 132 of the insulating cover 130.

Therefore, according to a preferred embodiment of the present invention, as shown in Fig. 4, it is preferable that the electrode catalyst layer 120 may be formed of an electrode catalyst layer having a sufficient thickness t. For example, the electrode catalyst layer may be formed to have a sufficiently thick thickness of 20 µm to 3 mm, which is difficult to form by plating.

The electrode catalyst layer may be adhered to or attached to the facing surface 110s of the conductive body 110, and the electrode catalyst layer may be installed to be stacked by being placed on the facing surface 110s of the conductive body 110. Since the electrode catalyst layer is stacked without being adhered to the conductive body 110, the electrode catalyst layer can be easily separated from the conductive body 110 for reuse or recycle.

The electrode catalyst layer is formed to have a uniform thickness as a whole, but the present invention is not limited thereto. Since platinum has excellent ductility, it can be easily made into an electrode catalyst layer by rolling.

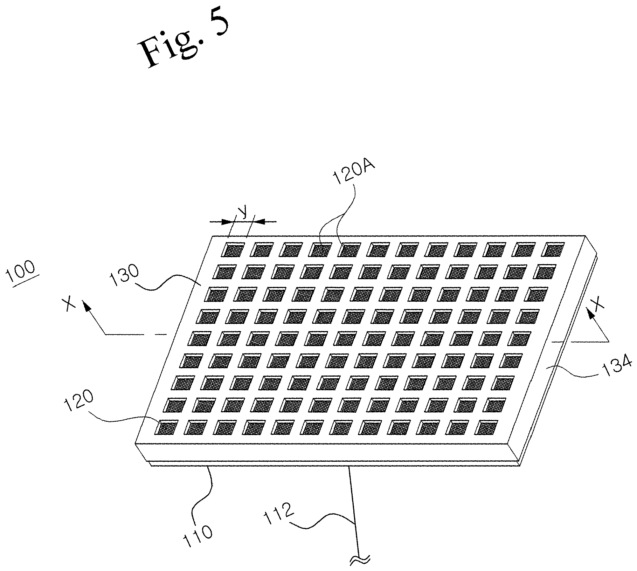

The insulating cover 130 is formed of a material that electricity cannot flow by conduction, and may be formed of, for example, any one of plastic, resin, polyurethane, and rubber. A plurality of through portions 132 are formed in the insulating cover 130 and are coupled to or fixed to the conductive body 110 with the electrode catalyst layer 120 interposed therebetween. As shown in Fig. 5, the electrode catalyst layer 120 is exposed to the outside through the through portions 132 of the insulating cover 130, and areas other than the through portion 132 are hidden from the outside by the insulating cover 130.

That is, the insulating cover 130 serves to fix the position of the electrode catalyst layer and serves to expose only the first region 120A of the electrode catalyst layer 120 forming plural electricity current paths by the through portions 132 to the outside.

The through portions 120A of the insulating cover 130 may be formed in various shapes, for example, may be formed in a lattice shape as shown in the drawings, and thus and the first region 120A in which the electrode catalyst layer 120 forming a plurality of electricity current paths is exposed by the through portions 132 formed between the lattice. When the insulating cover 130 is formed in a lattice shape, as the first region 120A is accurately disposed at a predetermined interval, the interval between the plurality of electricity current paths p are kept constant, so that the constitution can maximize the efficiency of uniformly generating sterilizing components by electrolysis between the first electrode 100 and the second electrodes 100'.

Although the through portions 132 as forms of squares of the insulating cover are illustrated in the drawings, according to another embodiment of the present invention, the through portions 132 of the insulating cover 130 may be formed in any one of circular, elliptical, and polygonal shapes or their combinations.

As shown in Figs. 4 and 5, the insulating cover 130 is configured such that the outer extension member 134 is fitted to the conductive body 110 with surrounding the outer surface of the conductive body 110. That is, the insulating cover 130 may be detachably combined with the conductive body 110 by fitting. According to another embodiment of the present invention, several holes are formed in the insulating cover 130 so that the insulation cover 130 may be joined with the conductive body by 110 by bolts or screwing to the female threaded hole (not shown in the drawing) of the conductive body 110. Here, it is preferable that the insulating cover 130 is combined with the conductive body 110 such that the whole bottom surface of the insulation cover 130 is in contact with the outer surface of the electrode catalyst layer 120.

That is, the insulating cover 130 and the conductive body 110 may be formed in various shapes to be separable from each other, and are not limited to the shapes shown in the drawings.

Accordingly, as the electrode catalyst layer is not attached to the facing surface 110s of the conductive body 110 but is placed in a stacked form, when all of the platinum in the first region 120A forming the electricity current paths that is exposed to the outside is used completely, the insulating cover 130 is separated from the conductive body 110 and the electrode catalyst layer in the area other than the consumed first region simply collected, and platinum in the area other than the first region may be reused. Since platinum has very good ductility, it can be rolled into a new electrode catalyst layer of original thickness by adding new platinum to the collected electrode catalyst layer. Or, a new electrode catalyst layer may be shaped without adding platinum by rolling forming. Then, the reshaped new electrode catalyst layer is reused for the sterilizing electrode.

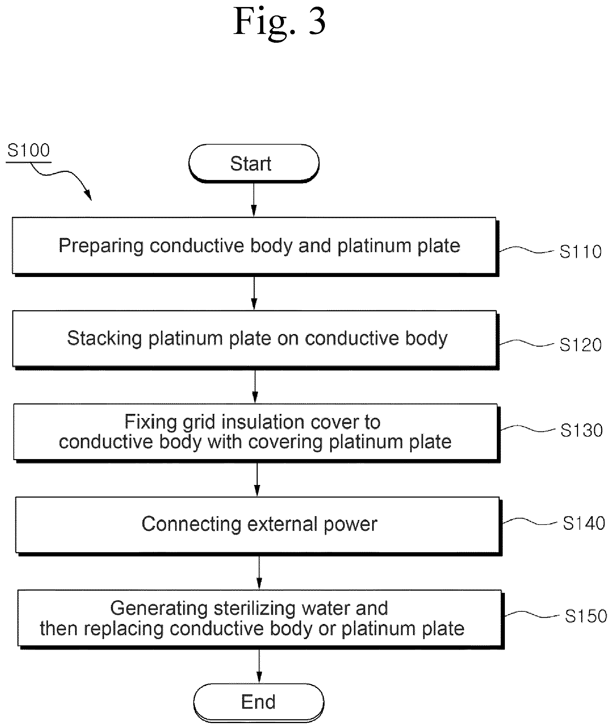

The sterilizing electrode 100 according to the present invention configured as described above may be manufactured according to the flow chart shown in FIG. 3.

Firstly, as shown in Fig. 4, a conductive body 110 formed of an electrically conductive material such as titanium and an electrode catalyst layer having a predetermined thickness are prepared (S110). Herein, the conductive body 110 has a facing surface 110s.

Then, the electrode catalyst layer is stacked and mounted on the facing surface 110s of the conductive body 110 in a non-adhesive manner (S120).

Then, an insulating cover 130 made of an insulating material in which a plurality of through portions 132 are formed is prepared, and the insulating cover 130 is fixed to the conductive body 110 in a state that the electrode catalyst layer is interposed between the conductive body 110 and the electrode catalyst layer 120 (S130). Accordingly, as shown in Fig. 5, the first region 120A of a part of the electrode catalyst layer is exposed to the outside through the through portions 132 of the insulating cover 130. Here, it is preferable to detachably fix the insulating cover 130 to the conductive body 110.

Then, as shown in Fig. 6, after the first region 120A of the first electrode 100 and the first region 120A of the second electrode 100' are aligned to face each other, electric DC current is supplied from the external power supply 200 to the conductive body 110 of the first electrode 100 and the second electrode 100'. However, the present invention is not limited to a configuration in which the first electrode 100 and the second electrode 100' are aligned each other, according to another embodiment of the present invention, the first regions 120A of the first electrode 100 and the first region 120A of the second electrode 100' may be arranged in a form that they completely face each other with a certain height difference.

Accordingly, the electricity current paths p are formed between the first electrode 100 and the second electrode 100, through which electric charges move between the first regions 120A exposed by the through portions 132 of the insulating cover 130. Here, the electricity current paths p are formed to maintain predetermined distances with one another by the predetermined distances between the through portions 132 of the insulating cover 130. Therefore, the electrolysis in the electricity current paths is made uniformly, Therefore, the electrolysis in the current paths are made uniform, and oxidants such as hypochlorous acid HOCl, OH radicals, and hydrogen peroxide are generated in the liquid 55 as a sterilizing component, so that the liquid can be changed into sterilizing water. At the same time, it is possible to obtain an effect of generating sterilizing components having a high sterilization capability while minimizing the power consumption and platinum consumption.

For this purpose, it is preferable that the liquid contains a chlorine Cl component. In addition, since hypochlorous acid is harmless to the human body and has a high sterilizing capability, in order to increase the generated amount of hypochlorous acid, the pH value of the liquid is preferably in the range of 3.0 to 8.0.

The sterilization apparatus 1 shown in Fig. 6 includes a set of sterilizing electrodes 100 and 100' with a sufficiently thick electrode catalyst layer, and thus, when a high current is applied thereto, sterilizing components are efficiently and continuously generated on the electricity current paths p between the first region 120A of the set of sterilizing electrodes 100 and 100' for a long time.

However, when the process of manufacturing sterilizing water for a long time, the electrode catalyst layer 120 in the first region 120A exposed to the liquid by the through portions 132 of the insulation cover 130 is consumed. When the electrode catalyst layer is consumed to a reference level, the electrode catalyst or the insulation cover 130 is replaced (S150).

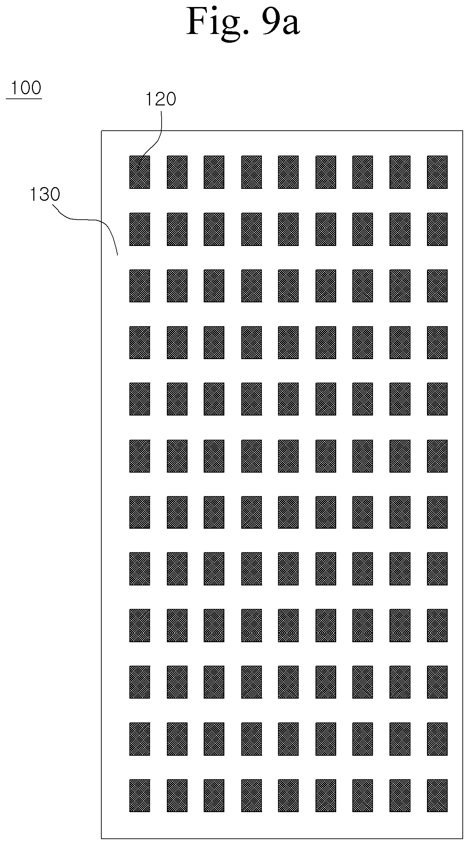

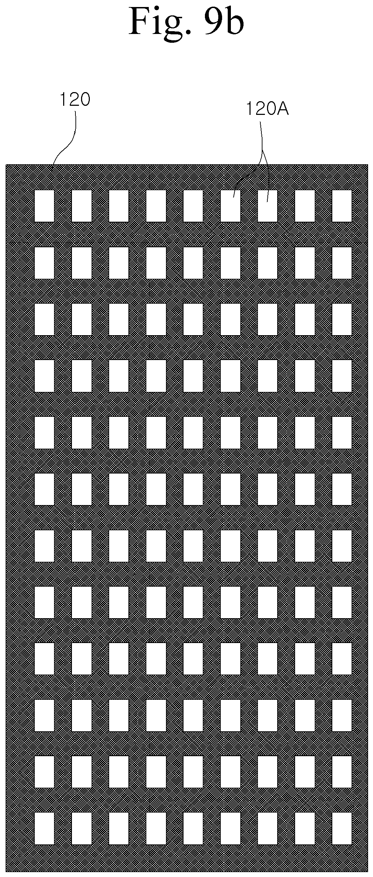

That is, as shown in Fig. 9A, the first electrode 100 constituting the sterilizing electrode has a first region 120A through which platinum is exposed by the through portions 132 of the insulating cover 130. However, when the use time of the sterilizing electrode reaches the reference value, it is inevitable that platinum in the first region 120A is almost exhausted so that the normal electrolysis may not be realized. Therefore, when the insulating cover 130 is separated from the conductive body 110, as shown in Fig. 9B, the platinum in the first region 120A is almost all consumed while platinum in the area other than the first region 120A maintains its initial thickness.

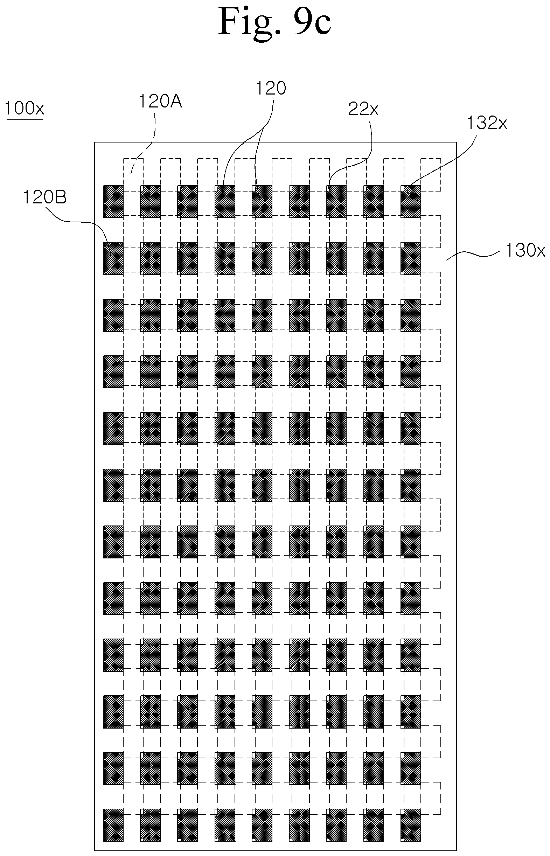

Accordingly, as shown in Fig. 9C, the other insulating cover 130x in which the through portions 132x are arranged in a different arrangement from the through portions 132 of the insulation cover 130 shown in Fig. 9A is combined to the conductive body 110. Then, the first region is changed into positions indicated as "120B" different from the first region 120A of Fig. 9A. Accordingly, by replacing insulating cover 130, the first electrode 100 can be reused without replacing the electrode catalyst layer as the electrode 100x of Fig. 9C

On the other hand, a large amount of the electrode catalyst layer 120 in the first region 120A shown in Fig. 9B is consumed, and also the new first region 120B in the electrode 100x shown in Fig. 9C is also consumed and becomes thinner when the usage time elapses. By the way, as the electrode catalyst layer is not adhered to nor plated on the facing surface 110s of the conductive body 110, but is stacked on the facing surface 110s of the conductive body 110, it is very easy to separate the catalyst layer from the conducting body 110 after separating the insulating cover 130 from the conductive body 110. Accordingly, as the electrode catalyst layer in which the first region has been consumed is reused to manufacture a new electrode catalyst layer, it is possible to reuse all of the expensive platinum without discarding even a little thereby obtaining an advantage of increasing economic efficiency.

Accordingly, when the consumed amount of platinum in the first region 120A exceeds the reference value, the electrode catalyst layer or the insulating cover may be replaced so that the sterilizing electrode can be used semi-permanently.

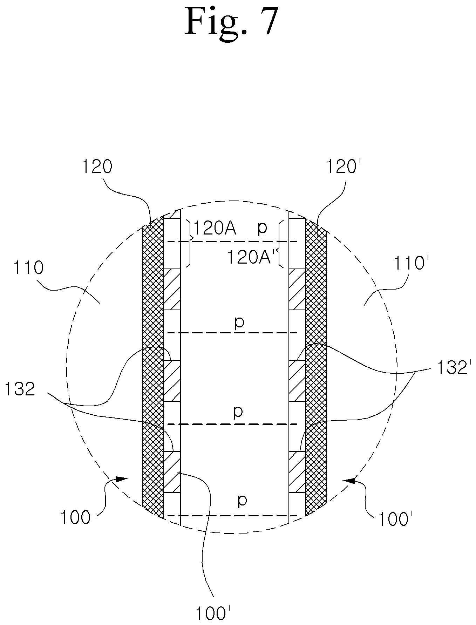

Meanwhile, according to an embodiment of the present invention shown in Figs. 6 and 7, both the first electrode (positive electrode) and the second electrode (negative electrode) constituting the sterilization apparatus 1 comprise conductive bodies 110 and 110, electrode catalyst layers 120 each of which is formed as a thin plate and insulation cover 130 wherein electricity current paths p are formed between the first region 120A, 120A' of the electrode catalyst layers exposed by the through portions 132 of the insulation cover 130.

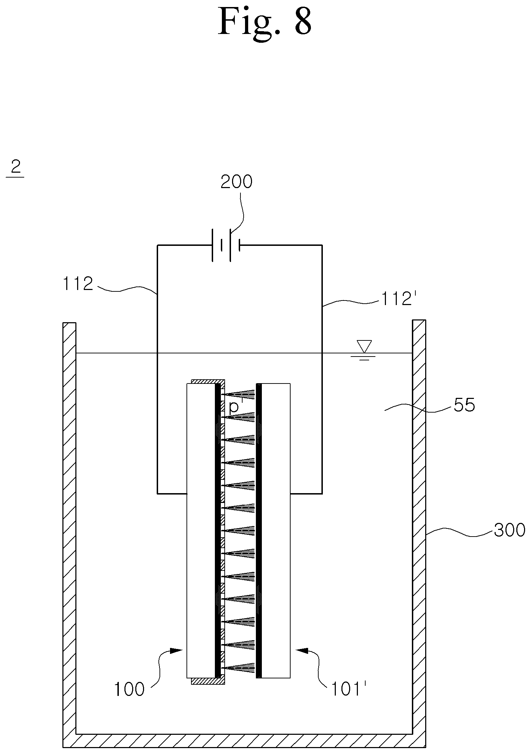

According to another embodiment of the present invention, the first electrode 100 constituting the sterilization apparatus 2 is configured as shown in Figs. 4 and 5, but the second electrode 101' may be configured as shown in Fig. 8 without an insulating cover. In this case, the electricity current paths p' are formed as spreading shapes instead of line shapes so that electric charges spreadly move from the first region 120A of the first electrode 100 to the second electrode 101'. Compared to the configurations shown in Figs. 6 and 7, the efficiency of generating the sterilizing component is lowered. However, as staring points of the electricity current paths p' are positioned with one another partitioned by the insulating cover 130 of the first electrode 100, the advantage of high efficiency of generating sterilizing components can be obtained compared with the configuration which both electrodes are formed as simple plate shapes.

Fig. 8 illustrates a configuration in which an electrode catalyst layer is formed on the second electrode 101', but according to another embodiment of the present invention, the electrode catalyst layer may not be formed on the second electrode 101'. Meanwhile, in Fig. 8, a configuration in which the positive electrode forms the first electrode 100 of Figs. 4 and 5 while the negative electrode is an electrode 101' without an insulating cover is illustrated, but according to another embodiment of the present invention, the negative electrode may form the first electrode 100 of Figs. 4 and 5, and the positive electrode may form the electrode 101' without an insulating cover.

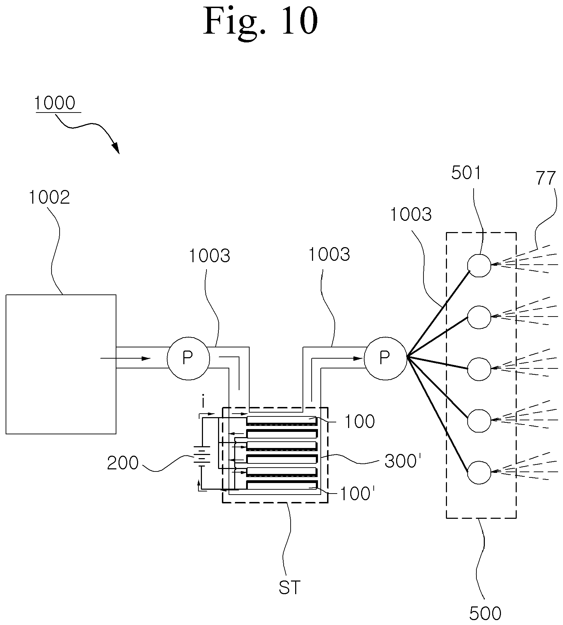

On the other hand, the sterilization apparatus 3 according to another embodiment of the present invention using the sterilizing electrode 100 manufactured as described above, as shown in Fig. 10, comprises: at least one set of sterilizing electrodes 100 and 100' installed in the Sterilization area ST that is at least partially immersed in liquid' a liquid supply pipe 1003 serving as a conduit through which liquid is transferred from the liquid supply unit 1002; and a sterilizing water supply unit 500 for supplying to the intended uses sterilizing water containing sterilizing components in the sterilization area ST.

Here, the electrodes shown in FIG. 5 may be used for the sterilizing electrodes 100 and 100'.

The liquid supply unit 1002 may be formed as a liquid storage tank, and the liquid is transferred to the sterilized water supply unit 500 through the sterilization area ST using the pump P disposed at the liquid supply pipe 1003. It is preferable that the liquid supplied from the liquid supply unit 1002 contains a chlorine component, and may be water whose chlorine content is separately adjusted by administering salt, or may be applied as universally used water such as tap water.

If necessary, liquid may be supplied to the sterilization area ST, and sterilization components are generated in the sterilization area ST by the sterilizing electrodes 100 and 100' in a state where the flow of the liquid is stopped and then the water in the sterilization area ST is controlled to flow and be transferred to the water supply unit 500. However, as the sterilizing electrodes 100 and 100' according to the present invention are formed to have a thickness of several tens to several thousand times compared to the prior art, as the electrode catalyst layer 120 may produce more sterilizing components with supplying high current, sterilizing water can be manufactured and supplied in real time even in a state in which liquid is continuously supplied.

On the other hand, as shown in Fig. 10, the electrode receiving container 300' in which the set of sterilizing electrodes 100 and 100' are accommodated is disposed at a position between bent area in the lower direction of the liquid supply pipe 1003 and may be defined as a sterilization area ST having a wider flow cross-section than the flow cross-section of the liquid supply pipe 1003.

Accordingly, even if the set of sterilizing electrodes 100 and 100' are installed in the liquid flow field to produce sterilizing water in real time, the flow rate of the liquid passing through the sterilization electrodes 100 and 100' is lowered in the sterilization area ST. Thus, as a larger amount of liquid passes through the space between the sterilizing electrodes 100 and 100' per unit time, sterilizing water containing more sterilizing components such as OH radicals and hypochlorous acid generated by the sterilizing electrodes 100 and 100' Can be supplied to the sterilized water supply unit 500.

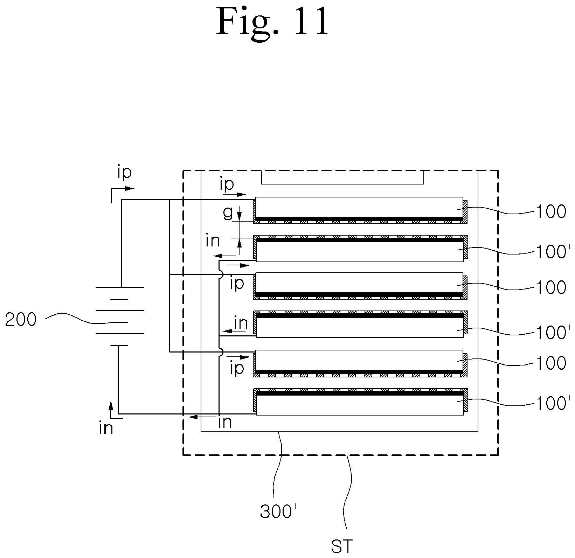

As shown in Fig. 11, the sterilizing electrodes 100 and 100' include a first electrode 100 supplied with a positive current ip from the power supply 200, and a negative electrode 100' supplied with a negative current in from the power supply 200. The first electrode 100 and the second electrode 100' form a set as a pair, and each of the electrode catalyst layers 120 of the first region 120A exposed by the respective through portions 132 are aligned to face each other.

Here, the external power supply 200 is made of a direct current power source, and the current supplied to the first electrode 100 and the second electrode 100' is determined to be approximately 10A or more, for example, a size of 30A to 40A may be used. Here, the supply voltage may be variously determined, and a high voltage of 3V to 500V may be maintained, for example.

Even if a large amount of sterilizing components such as hypochlorous acid, hydrogen peroxide, and ozone are generated in the liquid by an electrolysis with continuously supplying such a high current, as the electrode catalyst layer 120 is much thicker than that formed by the conventional plating layer such as a thickness of 20 µm to 3 mm, sterilizing water having high sterilizing capability can be prepared by generating a large amount of sterilizing components per unit time for a long time.

Moreover, as the first regions 120A and 120A' of the first electrode 100 and the second electrode 100' are installed in a position facing each other, electric charges are apt to be concentrated on the first regions 120A and 120A' which are not covered by the insulating cover 130. Thus, as the electric charges are gathered on the first regions 120A and 120A', the electrolysis may be generated mainly in electricity current paths p between the first regions 120A and 120A' thereby minimizing the lost electric charges which are not involved in electrolysis, generating a large amount of sterilizing components and increasing power use efficiency.

Figs. 10 and 11 illustrate a configuration in which the sterilizing electrodes are installed in the sterilization area ST where the flow of the liquid is slowed instead of providing a separate sterilization area. That is, at least one set of sterilizing electrodes are disposed along the supplying pipe, and the sterilizing components are generated in real time while liquid flows between the first electrode and the second electrode. In addition, the sterilizing water produced while the sterilizing components are generated in real time may be directly supplied to an object to be sterilized, or may be supplied in a state contained in a separate storage container.

Meanwhile, although drawings illustrate a configuration in which both the first electrode 100 and the second electrode 100' are provided with the first region 120A, according to another embodiment of the present invention, as shown in Fig. 8, one of the sterilizing electrodes 100 and 100' may be formed without the first region 120A as a flat surface.

Meanwhile, in the sterilization area ST in which the sterilizing electrodes 100 and 100' are disposed, a liquid may remain in a state or a liquid may flow. That is, even if the liquid supplied from the liquid supply unit 1002 through the liquid supply pipe 1003 is flowing in the sterilization area ST, the current applied to the sterilizing electrodes 100 and 100' in the liquid flow field is set high enough such as 10A or more, it is possible to create a larger amount of sterilizing components in a unit time, so that a large amount of sterilizing water can be produced in real time even in a flowing liquid flow field and can be supplied directly in a short time to the object such as livestock.

The sterilizing water supply unit 500 supplies sterilizing water containing sterilizing components generated by the set of electrodes 100 and 100' in various forms such as spraying on or spraying in a jet form to an object to be sterilized, cleaned, or disinfected.

On the other hand, as the time of use of the sterilization apparatus 3 goes on, the electrode catalyst layer 120 is consumed little by little and the gap between the electrode catalyst layers 120 of the first regions 120A and 120A' increases. Then, even if current is applied to the sterilizing electrodes 100 and 100' according to the strength of the applied current, a predetermined amount of sterilizing components is not generated.

When operating in such a state, the concentration of the sterilizing components in the sterilizing water 77 is lower than the predetermined value in normal operation, so that the sterilizing effect of the sterilizing water used for livestock houses or kitchen utensils cannot be obtained. Accordingly, the present invention operates by dividing into a first mode and a second mode in which the intensity of the current applied by the power supply unit 200 is adjusted differently from each other.

Firstly, in the first mode in which sterilizing components of a predetermined concentration is generated in the liquid surrounding the sterilizing electrodes, a first current of higher one such as between 10A to 50A is applied from the power supply 200 as described above, and a large amount of sterilizing components such as hypochlorous acid and OH radicals are generated per unit time and thus sterilizing water is produced.

In addition, prior to executing the first mode, a second current lower than the first current is applied from the power supply unit 200 to indirectly detect the used amount of the electrode catalyst layer 120. That is, when the amount of consumption of the electrode catalyst layer 120 increases, the distance between the electrode catalyst layer of the first electrode 100 and the electrode catalyst layer of the second electrode 100' is increased. If the distance between the electrode catalyst layer of the first electrode 100 and the electrode catalyst layer of the second electrode 100' increases, high current such as 10A or more applied to the electrodes 100 and 100' will make electricity current conduction between the electrodes 100 and 100' whereas low current such as 50mA to 500mA will not make electricity current conduction between the electrodes 100 and 100' thereby sensing the current conduction in accordance with the applied current value and detecting whether the residual amount of the electrode catalyst layer 120 is an appropriate amount for the manufacture of sterilizing water. Here, the second current in the second mode is preferably set to be 1/10 or less compared to the first current in the first mode.

In particular, as shown in Fig. 11, as the electricity current paths between the electrodes 100 and 100' are formed only between the first region 120A of the electrode catalyst layer 120, it is possible to obtain an advantageous effect of greatly improving the accuracy of detecting the amount of use of the electrode catalyst layer 120 by detecting whether or not the electricity current paths between the electrode catalyst layers 120 is formed.

That is, as each of the sterilizing electrodes 100 and 100' has the first region with a plurality of positions to form the current paths, and as the electrode catalyst layer 120 is exposed in the first region 120A, the electric charges of the second current in the second mode are apt to be concentrated on the first region of the electrode catalyst layer 120. Therefore, in case of a flat electrode, as charges are scattered, it is difficult to accurately detect the current state between the first electrode and the second electrode. However, in case of the present invention, as the amount of the electric charges concentrated on the first region 120A and 120A' is always constant under the predetermined current supply in the second mode, it is possible to accurately detect the residual amount of the platinum body based on whether the electricity current paths are formed between the first electrode and the second electrode when the second current is applied in the second mode.

Here, the magnitude of the second current may be determined as the threshold current value at which the electricity current paths are not formed when the electrode catalyst layer 120 are used for the lifetime and consumed, and at which the electricity current paths are formed when the electrode catalyst layer 120 are not used for the lifetime, by taking into account the strength of the first current in the first mode, the type of liquid, and the distance between the sterilizing electrodes.

Through this, prior to executing the first mode of generating sterilizing components in the liquid, the second mode of applying the second current of the low threshold value to the first electrode and the second electrode is executed, and the residual amount of the electrode catalyst layer 120 is checked whether it is suitable for executing the first mode, whereby it is possible to obtain an advantageous effect of ensuring the reliable production of sterilizing components.

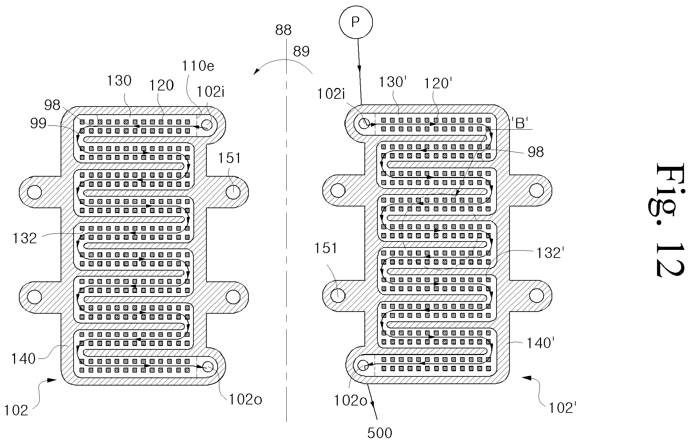

On the other hand, according to another embodiment of the present invention shown in Figs. 12 to 13, instead of immersing the electrodes in a still liquid with an electrolytic material for generating sterilizing components by electrolysis, a flow path 98 of a liquid with a dissolved electrolytic material such as chlorine is formed between the first electrode 102 and the second electrode 102', and it is configured to produce a certain amount of sterilizing components by the electrolysis of the liquid which is passing between the first electrode 102 and the second electrode 102'.

More specifically, each electrode 102 and 102' to which DC power is supplied from a power source (not shown) includes a conductive body 110 and 110', an electrode catalyst layer 120 stacked on the facing surface of the conductive body 110 and 110', an insulation cover 120 and 120' stacked on the outer surface of the electrode catalyst layer 120 and having through portions 132 and 132' to expose the part of the electrode catalyst layer 120 to the flow path 98, and a spacing member 140 stacked on the insulating covers 130 and 130' to form a serpentine flow path 98 between the electrode catalyst layers 120 and 120'. A set of sterilizing electrodes is manufactured by superimposing each of the electrodes 120 and 120' of Fig. 12 in the direction indicated by reference numeral 89 with respect to the center line 88.

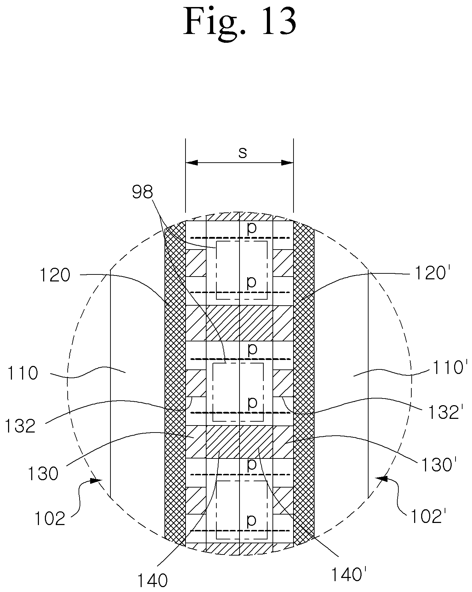

Here, the spacing member 140, as shown in Figs. 12 and 13, play roles in forming a gap s between the insulating covers 130 and 130', in maintaining the gap s between the electrode catalyst layers 120 and 120' at a predetermined interval, and in forming a zigzag flow path 98 between the insulating covers 130 and 130'. The spacing member 140 may be formed of an electrically conductive material or may be formed of an insulating material. In the drawings, the spacer member 140 is formed as a separate member from the insulating covers 130 and 130', but according to another embodiment of the present invention, the spacer member 140 may be integrally formed by the insulating cover 130, 130'.

Each of the electrodes 102 and 102' facing each other is coupled to each other into a single body by fastening bolts through holes 151 of the flange portion. Here, the power supplied from a power source (not shown) supplies positive power and negative power to the first electrode 102 and the second electrode 102'.

In addition, an inlet 102i through which liquid is introduced is formed at one end of the flow path 98 formed by the spacer member 140, and the liquid is discharged at the other end of the flow path 98 formed by the spacer member 140. While power is supplied to the conductive bodies 110 and 110', the liquid introduced through the inlet 102i passes through the flow path 98. when the liquid is discharged to the outlet 102o, the liquid is discharged as a sterilizing water containing a predetermined amount of sterilizing components, and thus may be supplied to the sterilized water supply unit 500.

According to another embodiment of the present invention, the conductive bodies 110 and 110', the electrode catalyst layers 120 and 120' and the insulating covers 130 and 130' may be disposed in various ways at a predetermined distance from each other so that a flow path may be provided between them.

With the above configuration, as the electrode catalyst layers 120 and 120' of the first electrode 102 and the second electrode 102' are spaced apart from each other at a predetermined interval s, similar to the above-described embodiments, the first region 120A is formed by the through portions 132 and 132' of the insulation cover 130 and 130' and the electricity current paths p are provided between the first regions 120A of the first electrode 102 and the second electrode 102' whereby the sterilizing components are uniformly generated in the liquid passing through a zigzag path between the first electrode 102 and the second electrode 102'.

Under such a configuration, the first electrode 102 and the second electrode 102' have a dimension of 88.5 mm^{∗}46 mm, platinum Pt was used for the electrode catalyst layer 120 of the first region exposed by the through portions 132 and 132', the distance s between the first electrode 102 and the second electrode 102' was set to 4 mm, and a 0.9% concentration saline solution was used as a liquid was set to flow through the flow path 98 at flow rate of 4.7 ml/sec. DC current of 100mA was supplied to the electrodes 102 and 102' for 4.9 seconds, and a pause of 0.1 second passed. And then it was continuously repeated that DC current of 100mA was supplied to the electrodes 102 and 102' for 4.9 seconds with a pause of 0.1 second. The concentration of hypochlorous acid in the liquid discharged from the outlet 120o was measured in units of hours.

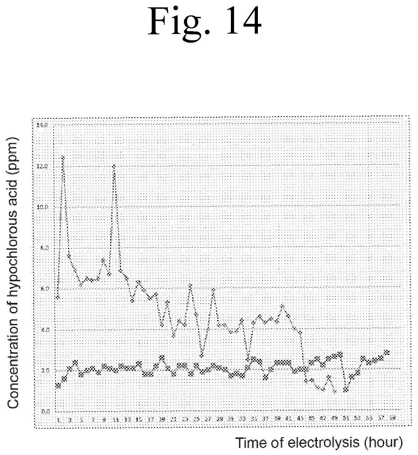

As a result, as shown in the lower measurement value (square shape) of the experimental data graph shown in FIG. 14, the concentration of hypochlorous acid in the liquid discharged through the flow path 98 between the electrodes 102 and 102' was measured in the range of 1.6ppm to 2.4ppm regardless of the passage of time, and the dispersion value was about 2.0ppm which were not large enough.

Meanwhile, as a comparative example, the first electrode and the second electrode having flat surface without the first region have the same dimensions as 88.5 mm ^{∗} 46 mm, platinum Pt was equally used for the electrode catalyst layers 120 and 120', the distance s between the first electrode and the second electrode was equally set to 4 mm, and a 0.9% concentration saline solution is equally used as a liquid was set to flow through the flow path 98 at the same flow rate of 4.7 ml/sec. DC current of 100mA was supplied to the electrodes and for 4.9 seconds, and a pause of 0.1 second passes. And then it was continuously repeated that DC current of 100mA is supplied to the electrodes for 4.9 seconds with a pause of 0.1 second. The concentration of hypochlorous acid in the liquid discharged from the outlet 120o was measured in units of hours.

As a result, as shown in the measured value (circular shape) on the upper side of the experimental data graph shown in Fig. 14, the concentration of hypochlorous acid in the liquid discharged through the flow path 98 between the electrodes initially had a high value of 5.8ppm to 12.3ppm, then gradually decreased with the passage of time, and tended to decrease to a value of 2.4ppm to 6.1ppm after approximately 24 hours. The concentration variation of hypochlorous acid was also large. It means that the electrolysis is irregularly performed between the flat electrode without the through portions 132, the amount of sterilization components produced is irregular. Also, when the flat electrodes are used for a long time, even if electrolysis is generated for the flowing liquid, foreign substances increase on the surface of the electrodes, and thus the concentration of the sterilizing components gradually decreases. In addition, it shows the problem that, as the consumption amount of the electrode catalyst layer is irregular, even when the electrode catalyst layer is not sufficiently consumed, the electrodes should be replaced with new ones.

To the contrary, in the present invention, the electrode catalyst layers 120 and 120' are formed sufficiently thick, and a plurality of electricity current paths p are separately formed with one another between the electrode catalyst layers 120 and 120' exposed by the through portions 132 of the insulation cover 130, thereby generating a uniform amount of sterilizing components in each current path p during the electrolysis. Thus, the concentration of the sterilizing components can be more precisely controlled and thus maintaining the sterilizing components of a uniform concentration for a long time, and further, it is possible to obtain an advantageous effect that the sterilizing water produced in accordance with the present invention can be used without irritation for a wider variety of uses.

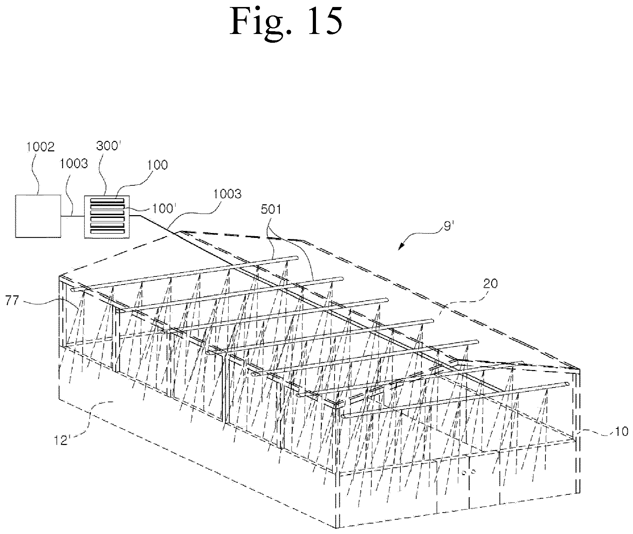

The sterilization apparatus 3 configured as described above can be used for various purposes. When the sterilizing water 77 supplied by the sterilizing water supply unit 500 is installed inside the barn in a spray form, as shown in Fig. 15, the sterilization apparatus may be applied for livestock barn sterilizer.

That is, the supply bar 501 provided with a plurality of spraying ports under the ceiling inside the barn may be arranged in a plurality of rows, so that the livestock sterilization apparatus may be configured to periodically spray the sterilizing water. Through this, not only the closed-type barn 9 of Fig. 1 but also the open-type barn 9' of Fig. 2 can be sterilized by the sterilizing water 77 which is sprayed in the air inside of the barn. By periodically sterilizing the pathogens or microorganisms carried in the air from the outside, it is possible to realize a clean and hygienic livestock environment.

In addition, the sterilizing water supply unit 500 may be provided in that sterilizing water may pass though the liquid transfer pipe of diverse objects such as such as tooth cleaners, water pipes, bidets, water purifiers, kitchen utensils, shower facilities, etc. whereby the sterilization apparatus can be used as for sterilizing and disinfecting the inside of the liquid transfer pipe

Moreover, it is possible to kill pathogens such as microorganisms and bacteria by spraying the sterilizing water produced by the sterilization apparatus 3 onto various objects such as kitchen utensils at high pressure. And, kitchen utensils may be washed by the sterilizing water, it is possible to kill pathogens remaining in kitchen knives or tableware through the same operation as a general dishwashing work thereby obtaining cleaner kitchen utensils sterilized in addition to washed.

As described above, in the present invention, an operation of producing a large amount of sterilizing water within a unit time can be continued for a long time. Also, sterilizing water having sterilizing components of a certain concentration range is continuously produced for a long time by electrolysis between the first regions 120A facing each other, thereby disinfecting livestock barns using a large amount of sterilizing water or disinfecting liquid transfer pipes or other various devices.

In particular, without any additional equipment before producing sterilizing water, the present invention enables to detect the consumed amount of a thick platinum plate in a second mode in which a lower current is applied to the electrodes compared with the first mode, so that the production of sterilized water having sterilizing components can be achieved for a long time thereby obtaining the advantageous effect of preventing the operation error in advance.

The above-disclosed subject matter is to be considered illustrative and not restrictive, and the appended claims are intended to cover all such modifications, enhancements, and other embodiments, which fall within the true spirit and scope of the present invention. Thus, to the maximum extent allowed by law, the scope of the present invention is to be determined by the broadest permissible interpretation of the following claims and their equivalents, and shall not be restricted or limited by the foregoing detailed description.

That is, in the drawings, several configurations have been described as an example, in which the first regions of the electrode catalyst layer in both the first electrode and the second electrode are exposed through the through portions of the insulating cover. However, the present invention is not limited thereto, and includes a configuration that the first region of the electrode catalyst layer is exposed in one of the first electrode and the second electrode through the through portion of the insulating cover, while the flat surface of the electrode catalyst layer is all exposed to outside in the other of the first electrode and the second electrode.

## Claims

1. A set of sterilizing electrodes including a first electrode and a second electrode disposed to face each other for generating at least one sterilizing component in a liquid containing chlorine between the first electrode and the second electrode by supplying power to the first electrode and the second electrode,
wherein at least one of the first electrode and the second electrode comprises:
a conductive body including an electrically conductive material for being connected to the power source;
an electrode catalyst layer formed by stacking electrode catalyst on a facing surface of the conductive body;
an insulating cover fixed to cover the outer surface of the electrode catalyst layer with the electrode catalyst layer interposed between the conductive body,
wherein a plurality of through portions are formed in the insulating cover so that a first region of a part of the electrode catalyst layer is exposed at a plurality of positions through the through portions and the remaining region of the other part of the electrode catalyst layer is covered.

2. The set of sterilizing electrodes of claim 1, wherein the electrode catalyst layer is formed of a sheet and is stacked on the facing surface of the conductive body without adhesion.

3. The set of sterilizing electrodes of claim 2, wherein a new electrode catalyst layer is capable of being replaced with the electrode catalyst layer when the first region of the electrode catalyst layer is used up.

4. The set of sterilizing electrodes of claim 2, wherein the thickness of the electrode catalyst layer is formed between 20 *µ*m and 3mm.

5. The set of sterilizing electrodes of claim 2, wherein the insulating cover is formed in grid shape, and the first region is defined by through portions formed between grids.

6. The set of sterilizing electrodes of claim 4, wherein the electrode catalyst layer is formed of a sheet and is stacked on the facing surface of the conductive body without adhesion.

7. The set of sterilizing electrodes of claim 2, wherein a new electrode catalyst layer includes a plated layer with the electrode catalyst on the outer surface, wherein the insulating cover is formed as grid shape, and wherein the first region is formed by the through portions formed between grids.

8. The set of sterilizing electrodes of claim 2, wherein the insulating cover is separably combined with the conductive body, and the electrode catalyst layer formed as sheet is separable from the conductive body.

9. The set of sterilizing electrodes of claim 8, wherein the insulating cover is combined with the conductive body by bolt fastening or fitting.

10. The set of sterilizing electrodes of claim 2, wherein the insulating cover is formed of at least one of plastic, polyurethane, rubber and resin.

11. The set of sterilizing electrodes of claim 1, wherein the insulating cover is replaceable with a new insulating cover with a plurality of through portions of which positions are different from those of the insulating cover.

12. The set of sterilizing electrodes of claim 1, wherein the conductive body is formed of at least one of titanium and copper.

13. The set of sterilizing electrodes of claim 1, wherein the electrode catalyst includes at least one of platinum, cobalt, iridium and nickel.

14. The set of sterilizing electrodes of claim 13, wherein the electrode catalyst is formed in a molecular structure in which any one of platinum, cobalt, iridium, and nickel is disposed in the center and graphene is coated on the surface thereof.

15. The set of sterilizing electrodes of claim 1, wherein both the first electrode and the second electrode comprise the conductive body, the insulating cover and the electrode catalyst layer;
and wherein the first region of the first electrode is aligned to face the first region of the second electrode.

16. The set of sterilizing electrodes of claim 15, wherein the sterilizing electrode set is used for manufacturing sterilizing water for livestock barns.

17. A method of manufacturing sterilizing electrode, comprising:
preparing a conductive body formed of electrically conducting material and has a facing surface;
stacking an electrode catalyst layer formed as a sheet having a predetermined thickness on the facing surface of the conductive body; and
fixing an insulating cover having a plurality of through portions with the electrode catalyst layer interposed between the conductive body;
wherein a part of the electrode catalyst layer is exposed to form a first region at plural positions through the through portions.

18. The method of manufacturing sterilizing electrode of claim 17, wherein the insulating cover is separably fixed to the conductive body.

19. The method of manufacturing sterilizing electrode of claim 17, wherein the insulating cover is formed as grid shape, and the first region is defined by through portions formed between grids.

20. A sterilization apparatus, comprising:
at least one of sterilizing electrode set in accordance with one of claims 1 to 15; and
a power supply for supplying electric power to the sterilizing electrode set;
wherein, when power is supplied to the sterilizing electrode set in the liquid containing the electrolytic, at least one of sterilizing component is generated in the liquid to make the liquid into sterilizing water.

21. The sterilization apparatus of claim 20, wherein both the first electrode and the second electrode comprise the conductive body, the insulating cover and the electrode catalyst layer;
and wherein the first region of the first electrode is aligned to face the first region of the second electrode.

22. The sterilization apparatus of claim 20, further comprising:
a supply part of supplying sterilizing water.

23. The sterilization apparatus of claim 22, wherein the supply part includes plural supply bars equipped with a plurality of spray ports are arranged in plural rows under the ceiling of the livestock barn.

24. The sterilization apparatus of claim 20, wherein the sterilizing electrode set is installed in the flow path through which the liquid flows.

25. The sterilization apparatus of claim 20, wherein the flow path is formed by a supply pipe for supplying the liquid, and the sterilizing water containing the sterilizing component is generated in real time by the sterilizing electrode set and is supplied through the supply pipe to an object.

26. The sterilization apparatus of claim 25, wherein the object includes at least one of tooth cleaner, bidets, water supply pipe for shower facilities, and kitchen utensils.
